# EUROPEAN PATENT SPECIFICATION

(11) **EP 2 554 653 B1**
(45) Date of publication and mention of the grant of the patent: **06.05.2020**
(21) Application number: 12461532.9
(22) Date of filing: 28.07.2012
(51) Int. Cl.: C12M 1/107

(54) **Method and system for transferring and mixing a biomass slurry in a hydrolyser and in a fermenter.**
Verfahren und System zum Übertragen und Mischen eines Biomasseschlamms in einem Hydrolyser und in einem Fermenter
Procédé et système de transfert et de mélange d'une suspension de biomasse dans un appareil à hydrolyse et fermentation

(30) Priority: 04.08.2011 PL 39586011
(43) Date of publication of application: 06.02.2013
(73) Proprietor: Instytut Chemii i Techniki Jadrowej, 03-195 Warszawa (PL)
(72) Inventor: Krylowicz, Adam, 22-400 Zamosc (PL); Usidus, Janusz, 22-400 Zamosc (PL); Chrzanowski, Kazimierz, 22-400 Zamosc (PL); Chmielewski, Andrzej, 04-802 Warszawa (PL)
(74) Representative: Belz, Anna

(56) References cited:
- EP-A1- 1 972 691
- DE-A1- 3 105 081
- GB-A- 2 204 056
- US-A- 5 773 526
- US-A1- 2003 059 927

## Description

The subject of the present invention is a method and a system for transferring and mixing a biomass slurry in a hydrolyser and in a fermenter.

Polish Patent No. 197595 discloses an invention entitled: "Method and system for the production of methane and electricity and heat", a method for the production of methane, especially from raw vegetable raw materials obtained from crops intended for that purpose. The method discloses recycling effluents produced in an individual process cycle for reuse in that cycle.

The monograph by Zbigniew Heidrich, Agnieszka Nieścior, published in Wodoci 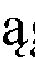 gi i kanalizacja nr 4 "Stabilizacja beztlenowa osadów ściekowych", Wydawnictwa Zarz 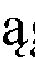 adu G 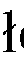 ównego Polskiego Zrzeszenia Inżynierów i Techników Sanitarnych Nr 759, Warszawa 1999, discloses a method for mixing sludge in a vertical fermenter using external pumps. Mixing the sludge in a fermenter using external pumps consists in taking a sediment from the bottom of a chamber and pumping it back to the upper part of the chamber, below sludge surface, thus facilitating the removal of scum formed on the surface of the fermenting sediment from the chamber".
The article published by Institut fur Energetik Und Umwelt GmbH: "Biogas production and use", training materials of the Renewable Energy Institute of EC BREC/IBMER, Warszawa 2009, discloses a method for mechanical mixing using paddles in a horizontal fermenter.
All these biomass mixing methods assist the process of releasing biogas from biomass slurry being hydrolysed and fermented, said biomass containing methane, carbon dioxide, hydrogen, hydrogen sulphide and small quantities of other gases. In a methanogenesis process, the following reactants take part: acetic acid, hydrogen and carbon dioxide.

The application EP 1972691 discloses process for the production of biogas by discontinuous, 2-step solids fermentation. The biomass is first pretreated to reduce the particle size to values below 10 mm in mixing tanks and grinder and substrate is transported to further tank where the hydrolysis will take place. The substrate is transferred into fermenters where the anaerobic digestion of the substrate will produce biogas. The application includes also device that includes elements for grinding, mixing, heating, fermenters and tank with bacterial cultures that stimulate the fermentation process.

The application DE 3105081 discloses a method for processing fibrous or non-fibrous materials, in particular horse manure, waste materials, excrement etc. which are subsequently fed to further processing. The material subsequently being repeatedly fed through the comminution device, fed back directly into the reservoir vessel after each comminution process and being continuously agitated there, and a device for carrying out the method.

For known and currently used biomass slurry mixing methods, especially in the vertical fermenters, the biogas obtained has methane content of 45% to 60%, mostly 54%.

According to the present invention, a method for transferring and mixing a slurry of biomass in water, hereinafter referred to as "biomass slurry" comminuted in a macerator comminuter, mixed with leachates from digested pulp separator, containing appropriate bacterial cultures, and again or repeatedly pumped through the macerator comminuter, in a hydrolyser and in at least one fermenter with the application of hydrolysis of biomass in the form of comminuted plants obtained especially from crops and/or organic waste in a hydrolyser and of an anaerobic process of processing the hydrolysed biomass to biogas in a fermenter, is characterized in that a biomass comminuted with about 10% of biomass on the dry weight basis is cyclically pumped to the hydrolyser until it is filled at 50-70%, after which the biomass slurry is mixed in a single of multiple subsequent hydrolyser zones using slurry streams formed and accelerated by single or multiple subsequent jet nozzles. During such mixing, this slurry hydrolyses in a known way. The slurry, now partially hydrolysed, is pumped in portions to a longitudinal fermenter with a bottom sloped at an angle of approximately 0,3 % to 0.9 % toward the outlet, causing the slurry to flow horizontally, and the slurry is subjected to zonal mixing using the slurry streams formed by jet nozzles in subsequent zones of the fermenter, counting from the first zone to the penultimate zone. The biomass slurry is continuously fed into the last zone of the fermenter in the form of a broadening conical stream formed in the fermenter biogas exit area. The fermented biomass slurry, after pumping out from the end of the fermenter, is divided into two streams. The first stream, in form of reflux, is fed in its first portion to the fermenter tank, in the second portion to the hydrolyser tank and in the third portion to the macerator comminuter tank. The second stream of the fermented biomass is fed to the digested pulp separator that separates leachates from concentrated digested pulp, and a part of digested pulp separator leachate is recycled back to the macerator comminuter tank and/or to the hydrolyser tank, and the biogas exiting the fermenter is subjected to purification and further processing in a known way.

The zonal mixing of biomass slurry, performed in a single or multiple zones, said zones subjected to a hydrolysis process in a hydrolyser, is characterised in that the biomass being hydrolysed is sucked in at the bottom of the hydrolyser and pumped to a jet nozzle in a single zone or to subsequent slurry jet nozzles that form and accelerate subsequently streams of biomass slurry, starting with the first zone at the inlet up to the end zone, said streams being ejected downward vertically to the surface of the slurry in the hydrolyser.

The zonal mixing in the fermenter of a biomass slurry being subjected to anaerobic processing into biogas, is characterised in that that the biomass is sucked in at the bottom of the fermenter in the first zone of the fermenter and pumped to the subsequent jet nozzles or to a first jet nozzle segment which forms streams of biomass starting from the first nozzle at biomass slurry inlet up to the last nozzle of that zone. The streams formed by the jet nozzles are directed upward, vertically to the surface of the biomass slurry in the fermenter. Next, the slurry stirring process is carried out in consecutive segments of the slurry mixing unit.

During the hydrolysis and fermentation, especially during the pumping of the biomass slurry from the hydrolyser to the fermenter, an equal, elevated pressure of gases in the hydrolyser and the fermenter is maintained.

Preferably, the biomass transfer and mixing processes are carried out in at least two hydrolysers arranged in parallel and. The larger amount of fermenters is due to difficulty to manufacture one tank of large capacity.

An advantage of the method of zonal transfer and mixing of biomass slurry in the hydrolyser and fermenter is that it causes scrubbing of CO₂ and H₂S from biogas by the streams of biomass slurry and recycling these gases together with biomass from the surface of the slurry in the fermenter to an anaerobic zone where biomass is processed to biogas, causing also breakage of the sludge being formed at the surface of the slurry. Another advantage is the horizontal movement of the biomass slurry area containing possibly harmful contaminants introduced from the outside with biomass to the hydrolyser in form of bacteriophages, compounds of copper, zinc, etc., along the horizontal hydrolyser and then the fermenter until the contaminated biomass leaves the fermenter. Such a method of transferring and mixing of biomass slurry in a hydrolyser and a fermenter increases methane content in biogas to 65% ÷ 80%, typically to 70% ÷ 75% - which was found experimentally at a pilot plant. Current vertical fermenters do not have such possibility to remove such a contaminated biomass slurry, and methane content in the biogas reaches a value of 40% ÷ 60%, mostly 54%. Recycling a part of reflux and a part of leachate containing appropriate bacterial cultures to the macerator comminuter tank and to the hydrolyser and to the starting zone of the fermenter results in less biomass consumption in the process of its hydrolysis and in the process of anaerobic processing of biomass to biogas, speeds up these processes and reduces water consumption. Sloping the fermenter toward its outlet helps to remove sand and other solid impurities from the fermenter.

These effects were obtained by comminution of biomass and mixing it with the recycled reflux and leachates containing appropriate bacterial cultures, as well as with water, then causing the slurry to move horizontally in the hydrolyser and in the fermenter, by recycling the reflux and leachate for reuse, and also by zonal mixing of the biomass slurry in the hydrolyser and the fermenter using slurry streams subsequently directed by jet nozzles vertically to the surface of the slurry in the hydrolyser and of the biomass anaerobically processed to biogas in the fermenter, as well as by the stream of slurry scrubbing CO₂ and H₂S from biogas in the final zone of the fermenter.

The subject of the present invention is also a system for transferring and mixing a biomass slurry in a hydrolyser and in a fermenter.

A system for transferring and mixing a biomass slurry in a hydrolyser and fermenter comprising, a tank with a feeder at its outlet that is connected through macerator comminuter, slurry pump and valve with the tank at its inlet and the pipeline with a valve and water pipeline, a hydrolyser, a fermenter, and a digested pulp separator are connected to the tank, is characterised in that the slurry pump is also connected, via a second valve, to the hydrolyser tank. The hydrolyser is equipped with a hydraulic mixing unit, and in the end zone of the hydrolyser, at hydrolyser bottom, there are suction nozzles arranged transversally with respect to the hydrolyser axis, said suction nozzles connected via a pump and a siphoned pipeline to a fermenter tank. The hydrolyser mixing unit consists of a mixing pump with a comminuting attachment, one end of said pump connected to suction nozzles located at hydrolyser bottom transversely with respect to the hydrolyser tank axis, and the other end of the pump connected to a control valve or a plurality of control valves, each control valve connected to a jet nozzle or a plurality of jet nozzles located in the upper part of the hydrolyser.

The fermenter tank is sloped at an angle of from 0,3 % to 0,9 % toward the outlet of the fermented biomass. The fermenter is equipped with at least one mixing unit and has suction nozzles in its end zone at the bottom, said suction nozzles positioned transversally with respect to the fermenter tank axis and connected to a pump that discharges the fermented biomass through two pipeline branches. Through the first pipeline branch, the fermenter is connected to the digested pulp separator, and through the second pipeline branch it is connected to the inlet side of the fermenter tank and the inlet side of the hydrolyser tank, as well as with the tank of the macerator comminuter.

The mixing segment of the fermenter comprises at least two jet nozzles, arranged transversally and/or longitudinally in relation to the axis of the fermenter tank, said jet nozzles placed in the upper part of the fermenter and connected to a slurry pump equipped with a comminuting attachment, said suction nozzles being placed at the bottom of the tank within the respective zone.

The fermenter tank comprises at its end part a biogas exit. Installed at the biogas exit is a head of a scrubbing device, said head connected through a pump to a number of suction nozzles located in the end part of the fermenter and positioned transversely to the tank axis.

A digested pulp separator comprises a concentrated digested pulp outlet and is connected via a leachate pipeline through valves and the second pipeline branch, to the hydrolyser tank, and through another valve and a pipeline it is connected to a leachate treatment facility.

The hydrolyser tank is connected via a gas conduit and gas valves to the fermenter tank which is equipped with a siphon to protect the tanks from excessive pressure.

Preferably the fermenter has two pumps at the outlet of the fermented biomass slurry. One pump is connected at one end to the suction nozzles located at the end of the fermenter tank, and at its other side, via a valve, to the reflux pipeline, the second pump being connected at one end to the suction nozzles located at the end of the fermenter tank, and at the other end via a valve and a pipeline to the digested pulp separator. This allows to separately route streams of fermented biomass in a given time. An example of a digested pulp separator is a decanter centrifuge.

Pipeline inlets at the tanks are provided with cut-off valves, allowing the replacement of damaged pipelines, valves and pumps without the necessity to evacuate the tanks.

The invention is further explained in examples of tranferring and mixing of a biomass slurry in hydrolysers and fermenters.

### Example I.

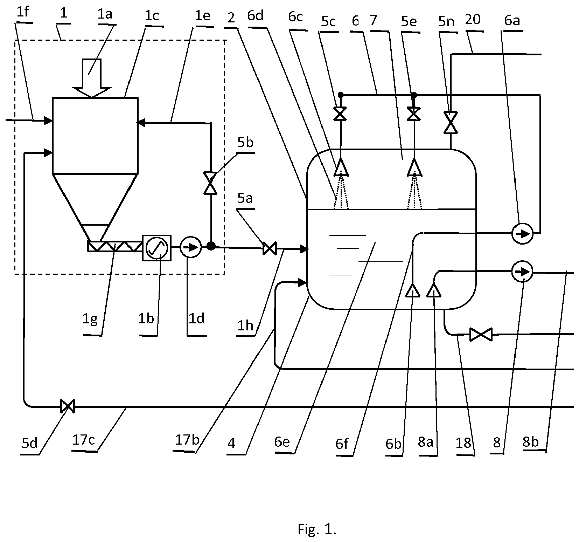

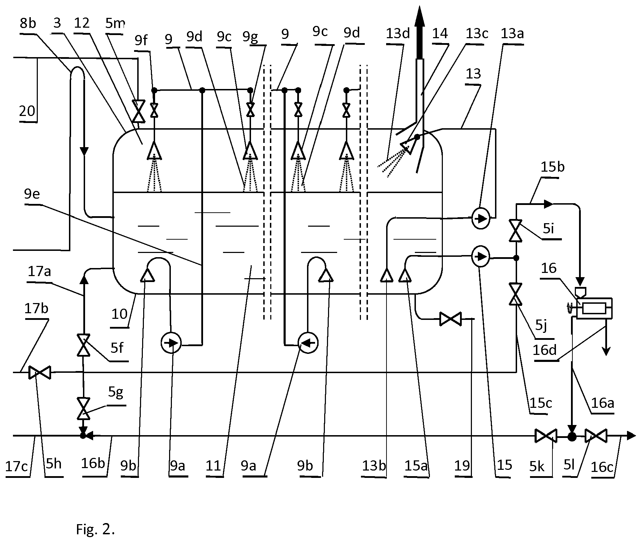

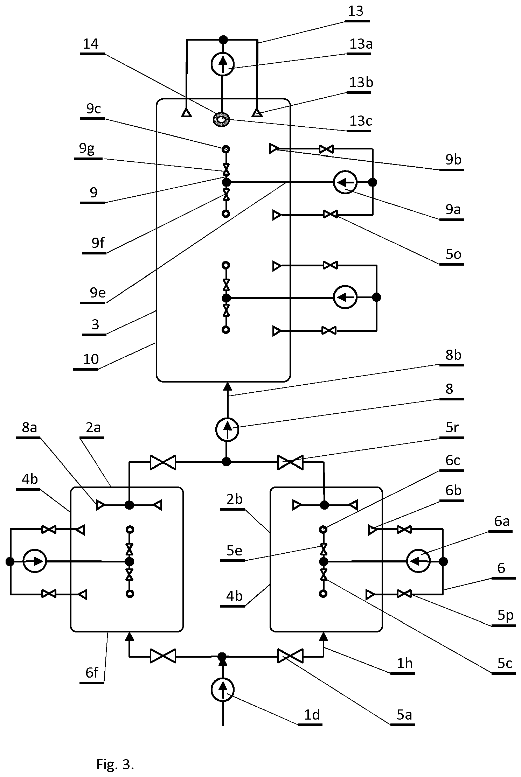

As the biomass for anaerobic biogas production a mixture of grass silage and maize silage **1a** was used, at a proportion of 1:1, and biogas was produced in fermenter tanks with volume of approximately 4000 m³ and two hydrolysers 2a and 2b with total volume of approximately 400 m³, as shown in Fig. 3; Fig. 3 uses references common with Fig. 1 and Fig. 2. Comminuted silage biomass particles were fed from a feed tank **1c** using a screw feeder **1g** to a macerator comminuter **1b** where they were further reduced in size and the biomass so comminuted was recycled back to the tank **1c**, and reflux with leachates and water were cyclically fed to the tank **1c** to form a slurry containing approximately 10% of biomass on the dry weight basis. The reflux and leachates recycled from the fermenter **3** and a centrifuge **16** contained suitable bacterial cultures involved in the process of hydrolysis of biomass in the anaerobic biomass production process. The biomass slurry passing through the macerator comminuter **1b** was pumped by a pump **1d** operating in a closed circuit until the required fineness was attained. The solution presented in this example employed two parallel connected hydrolysers **2a** and **2b.** The first hydrolizer **2a** had been filled up to 60% of its volume with fresh biomass slurry, and zonal mixing of the slurry was performed.

The biomass slurry being hydrolysed in the hydrolyser **2a** was zonally mixed in two zones by a mixing unit **6.** The zonal mixing of the biomass slurry in the hydrolyser **2a** consisted in that the slurry was taken at the bottom of the hydrolyser **2a** and pumped to a battery of jet nozzles **6c** located at the beginning of the hydrolyser **2a,** said jet nozzles forming slurry streams **6d** over the first mixing zone of the biomass **6e.** The biomass slurry streams **6d** were directed vertically to the surface of the liquid slurry **6e** in the tank **4a** of the hydrolyser **2a.** A control valve **5c** of the first nozzle battery **6c** was open, and another control valve **5e** was closed. The jet nozzles **6c** formed slurry jets **6d** mixing the biomass slurry **6e** in the first zone of the hydrolyser **2a.** The operating time of the first battery of jet nozzles **6c** was about 10 minutes. Then, the control valve **5c** of the first battery of jet nozzles **6c** was closed, and the second control valve **5e** of the second battery of nozzles **6c** forming slurry streams **6d** was open, and the mixing process in the second zone of the hydrolyser **2a** lasted 10 minutes. After that, both control valves **5c** and **5e** were closed, and the mixing pump **6a** of the hydrolyser **2a** was stopped. The process of zonal mixing of the biomass slurry in the hydrolyser **2a** was repeated twelve times a day. After the first hydrolyser was full, the second hydrolyser **2b** was filled with fresh biomass and the biomass slurry **6e** mixing process was carried out analogously like that in the hydrolyser **2a.** Biomass hydrolysis time in both hydrolysers **2a** and **2b** was 31 hours. Both hydrolysers **2a** and **2b** were connected in parallel and operated in an alternating sequence.

After the hydrolysis was completed, the slurry was fed in portions every two hours to the fermenter **3,** that is 12 slurry portions per day, until it was filled at 60%. One portion represented 1/12 of the volume of biomass in the hydrolyser **2a.** Next, portions of hydrolysed biomass together with portions of reflux accounting for 20% of the hydrolysed biomass were cyclically fed to the fermenter **3.** The time to pump through the biomass portion was 10 minutes, and the time interval to feed the next portion was 110 minutes. At the same time, the fermented biomass slurry was pumped out from the end of the fermenter **3** by a pump **15** so as to maintain unchanged the level of slurry **11** in the fermenter **3.** Such pumping of biomass slurry forced horizontal motion of the biomass **11** in the fermenter **3.**

In the course of movement of the slurry **11** through the fermenter **3** it was subjected to periodical mixing in successive zones. The mixing was started after pumping each portion of the hydrolysed biomass through to the fermenter **3.**

The mixing of the biomass slurry **11** in the first zone of the fermenter **3,** spanning over the first segment **9,** was carried out as follows. The biomass slurry **11** was taken at the bottom of the fermenter **3** by the suction nozzles **9b** located in the first mixing zone and, after a further comminution by the attachment of the pump **9a,** was pumped to the first battery of jet nozzles **9d** placed transversely to the axis of the tank **10,** at the beginning of the first mixing zone. A control valve **9f** was open to control the first battery of nozzles **9c** of the slurry **9d,** and a control valve of the second battery of nozzles **9c** of that segment **9** was closed. Accelerated jets **9d** were ejected from the nozzles **9c** and were directed vertically to the surface of the slurry **11,** causing its mixing. The jets **9d** also caused the breaking up of the sludge formed on the surface of the slurry **11** and scrubbing of CO₂ and H₂S from biogas, and also the movement of these gases together with biomass from the surface of the slurry **11** to an anaerobic area where biomass was processed into biogas. The operating time of the first battery of jet nozzles **9c** ejecting biomass slurry jets **9d** was around 4 minutes. Next, the control valve **9f** of the first battery of jet nozzles **9c** was closed and, after four minutes, another control valve **9g** was opened to control the operation of the second battery of nozzles **9c** forming slurry jets **9d** in the first mixing zone. The operating time of the second battery of jet nozzles **9c** forming biomass slurry jets **9d** was another 4 minutes, after which the control valve **9g** of the second battery of nozzles **9c** was closed and the first pump **9a** was stopped. The mixing time of the slurry **11** across the first segment **9** in the first zone was approximately 12 minutes. The biomass slurry **11** in the fermenter **3** was zonally mixed throughout consecutive segments **9** placed in series in successive mixing zones. The fermenter **3** was divided into five slurry mixing zones. In four zones of periodical mixing, segments **9** were used, each segment comprising two batteries of jet nozzles **9c.** The process of mixing of the biomass slurry **11** was started in consecutive zones until the fourth zone, and the time of ejecting jets from each subsequent jet battery **9c** was approximately 4 minutes. The process of zonal mixing of the slurry **11** across the segments **9** was repeated twelve times a day, and the total zonal mixing time of the slurry **11** in the fermenter **3** was approximately 12 hours. In the final part of the fermenter 3, the biogas formed was discharged, whereas in the biogas discharge area **14,** using a head **13c** a broadening stream **13d** was formed of the slurry **11** taken continuously from the end of the fermenter **3.** The biomass slurry stream **13d** scrubbed carbon dioxide and hydrogen sulphide from the biogas flowing out, which gases, having good solubility in water contained in the stream **13d,** were recycled to the anaerobic area where biomass was processed into biogas. The biogas leaving the fermenter contained 75% of biomethane, approximately 25% of carbon dioxide and small quantities of hydrogen sulfide. The biogas, after further treatment, especially removal of hydrogen sulfide, and after removal of water, was directed in 50% to feed a cogeneration unit producing electricity and heat, and the remaining biogas was directed to a biogas separator where it was separated into a stream of carbon dioxide and a stream of methane containing 96% of methane, which was sent to a tank. From the end zone of the fermenter the fermented slurry was cyclically taken and separated into two parts. The first part, approximately 50% of the fermented slurry, was fed through a pipeline branch **15b** to a decanter centrifuge **16,** and the other part of the slurry in the form of reflux was fed to the fermenter **3,** hydrolysers **2a** and **2b** and into the tank **1c** of the macerator comminuter **1b**.

The feeding of the reflux to the fermenter **3** was carried out in a continuous or cyclical mode, depending on the amount of biogas discharged. Leachates from the decanter centrifuge **16** were partially discharged to a waste treatment facility and partially transferred via a recycled leachate pipeline **16b** and fed, along with the reflux, to the tank of the macerator comminuter **1b**, and partly to hydrolysers **2a** and **2b.**

### Example II.

The below described process of transferring and mixing is illustrated in the drawing fig. 3, where references from drawings of figs 1 and fig 2 were used.

As biomass for anaerobic biogas production cereal straw and grass silage **1a** was used. Comminuted silage **1a** was mixed in the tank **1c** of the macerator comminuter **1b** together with the fermenter reflux, leachates and water, and was four times comminuted. The preparation of biomass for hydrolysis and feeding it to hydrolysers was carried out in a similar manner as in the first example. The solution presented in this example uses two parallel connected hydrolysers **2a** and **2b** of the total volume of 60 m³, and each of those hydrolysers was connected to the fermenter **3** with a volume of 600 m³. The slurry in the form of fresh biomass filling 60% of the hydrolyser **2a** volume was mixed in one zone of the mixing unit **6.** The mixing of the biomass slurry in the hydrolyser **2a** consisted in that the biomass slurry was taken at the bottom of the hydrolyser **2a** and pumped by pump **6a** with a comminuting attachment simultaneously to both jet nozzles **6c** located at the ceiling of the hydrolyser **2a** along the axis of the tank **4a.** Nozzles **6c** formed two slurry streams **6d** which were directed vertically to the surface of the slurry, mixing the slurry in the hydrolyser **2a.** The time of ejecting the streams **6d** was approximately 15 minutes. The mixing of the slurry was repeated twenty-four times a day. The adding of reflux and leachates to the tank **1c** of the macerator comminuter **1b** and to the tank **4a** of the hydrolyser **2a** was carried out similarly as in the first example. After the hydrolysis was completed, the slurry with a volume of about 18 m³ was fed every hour in portions of approximately 0.75 m³ to the fermenter **3**, making 24 portions of the slurry per day. The biomass hydrolysis process was also carried out in the second hydrolyser **2b,** so the average time of biomass hydrolysis in the two hydrolysers amounted to 31 hours. Both hydrolysers **2a** and **2b** operated in an alternating sequence. The hydrolysed biomass slurry was fed to the fermenter **3** until it was filled in 60%.

The process of anaerobic conversion of biomass **11** in the fermenter **3** lasted approximately 20 days. During the anaerobic process the slurry **11** was mixed in two successive zones. The mixing of the biomass slurry **11** in the first zone of the fermenter **3** consisted in that the slurry **11** was taken by suction nozzles **9b** located in the first mixing zone at the beginning of the fermenter **3** at its bottom, after which it was pumped by the pump **9a** equipped with a comminuting attachment simultaneously to both jet nozzles **9c** situated along the axis of the tank **10.** Using the jet nozzles **9c** two streams **9d** were formed which mixed the slurry **11** in the first zone. The streams **9d** also caused breakage of the sludge formed on the surface of the slurry **11** being mixed, as well as scrubbing of CO₂ and H₂S from the biogas above the slurry **11.** The operating time of the first slurry mixing segment **9** was about 5 minutes. Then the pump **9a** of the first slurry mixing segment **9** was stopped and after about 25 minutes the pump **9a** of the second fermenter segment was started and mixing of the slurry **11** in the second zone of the fermenter **3** lasted about 5 minutes. The process of zonal mixing of biomass slurry by segments **9** in the fermenter **3** was repeated twenty-four times a day. In the end zone of the fermenter **3,** the scrubbing device **13,** scrubbing CO₂ and H₂S from biogas and mixing the slurry **11,** was operated continuously. The operation of the scrubbing device **13** was carried out similarly to that in Example I.

The process ran in a continuous mode. After the fermenting of the slurry **11** was completed, a portion of hydrolysed biomass and a similar portion of reflux was introduced into the fermenter **3,** and at the same time a corresponding portion of fermented biomass was pumped out from the end of the fermenter **3** by a pump **15**. Half of the portion pumped out from the fermenter **3** was fed to a decanter centrifuge **16,** and the remainder of the fermented biomass was introduced to a reflux pipeline **15.** Circulation of the fermented slurry and leachates was similar to the circulation of those substances as in Example I.

The biogas leaving the fermenter contained approximately 70% of biomethane, 28% of carbon dioxide and small quantities of hydrogen sulfide. After further treatment, especially removal of hydrogen sulfide and water, the biogas was directed to supply a cogenerator producing electricity and thermal energy in cogeneration. The process of further purification and/or compression of biogas was carried out similarly as in Example I.

A system according to the present invention is illustrated in an embodiment in the enclosed drawing, where Figure 1 shows a schematic view of a biomass comminution and hydrolysis unit, Figure 2 shows a schematic view of a fermenter with a decanter centrifuge, Figure 3 shows the system composed of two hydrolysers and one fermenter, Figure 4 shows the system composed of two hydrolysers and two fermenters arranged in series, and Figure 5 shows a fragment of a fermenter with two fermented slurry pumps.

As shown in the drawing, preparatory unit 1 comprises screw feeder **1g** at the outlet of tank **1c**, which is connected via macerator comminuter **1b**, pump **1d** and valve **5b** to the inlet side of tank **1c**, and, additionally, pipeline **17c** and pipeline **1f** are connected to tank **1c**, while the pump **1d** is also connected via valve **5a** and pipeline **1h** from tank **4** of the hydrolyser **2.** In the bottom part of the tank **4** there are suction nozzles **6b** of mixing unit **6**, said nozzles connected via pipeline **6f,** pump **6a** equipped with comminuting attachment, and valves **5c** and **5e** that control two jet nozzles **6c** arranged in series along the tank **4** in its upper part. In the bottom part of the tank **4** of the hydrolyser **2** there are suction nozzles **8a** connected via pump **8** and pipeline with a siphon **8b** to tank **10** of the fermenter **3**. In the upper part of the tank **10** there are jet nozzles **9c** arranged in batteries, each battery composed of two nozzles **9c**, arranged in series along the tank **10** and batteries are grouped in segments **9**. In each of the segments **9,** heads **9c** are connected to pipeline **9e** through pump **9a** equipped with a comminuting attachment to suction nozzles **9b** located each in the bottom part of the tank **10** within a zone outlined by the span of the segments **9**. In the end part of the tank 10 there is a biogas exit with installed head **13c** of the scrubbing device **13**, said head connected via a pipeline and pump **13a** to suction nozzles **13b** located at the end portion of the tank **10**. In this part of the tank **10** there are also four suction nozzles **15a** situated transversally with respect to the axis of the tank **10**, connected to pump **15** from which there run two pipeline branches **15b** and **15c**. One pipeline branch connects the pump **15** via valve **5i** and pipeline **15b** to the decanter centrifuge **16**. The other pipeline branch connects the pump **15** via valve **5j** and pipeline **15c** to three sub-branches **17a**, **17b**, and **17c**, whereas the sub-branch **17a** is connected via valve **5f** to the beginning of the tank **10**, the second sub-branch **17b** is connected via valve **5h** to the beginning of the tank **4** of the hydrolyser **2**, and the sub-branch **17c** is connected via valves **5g** and **5d** to the tank **1c** of the macerator comminuter. The decanter centrifuge **16** is connected via pipeline **16a**, valve **5l** and pipeline **16c** to a leachates treatment facility, not shown in the drawing. The pipeline **16a** is connected via valve **5k,** pipeline **16b** and further via valves **5g** and **5h** as well as pipeline **17b** to the tank **4** of the hydrolyser **2**. The decanter centrifuge is also connected via the leachates pipeline **16a**, valve **5k**, pipeline **16b**, pipeline **17c** and valve **5d** to tank **1c**. The fermenter **3** is connected to the hydrolyser **2** through gas pipeline **20** via gas valves **5m** and **5n**.

Figure 3 shows a system composed of hydrolysers and a fermentor including biomass transfer and mixing equipment. The system is composed of two parallel arranged hydrolysers **2a** and **2b,** whose tanks **4a** and **4b** are connected to slurry pump **1d** via pipelines **1h** and valves **5a.** At their ends, the tanks **4a** and **4b** are equipped with suction nozzles **8a** located at the bottom of each tank **4a** and **4b**, situated transversally with respect to the axes of these tanks and connected via valves **5r** as well as pump **8** and pipeline with a siphon **8b** to the tank **10** of the fermenter **3**. Jet nozzles **6c** in the hydrolysers **2a** and **2b** as well as jet nozzles **9c** in the fermenter **3** are disposed along the axes of the tanks **4a** and **4b** and the tank **10**, in their upper parts. The suction nozzles **6b** of the mixing unit **6** of the hydrolyser **2a** and **2b** are connected via pipelines and valves **5p** as well as via pump **6a**, pipeline **6f** and control valves **5c** to jet nozzles **6c**. The suction nozzles **9b** of the mixing segment **9** are connected via pipelines and valves **5o** as well as via pump **9a**, pipeline **9e** and valves **9f** and **9g** to jet nozzles **9c**. At the end of the tank **10** of the fermenter **3** there is placed a scrubbing device **13** whose suction nozzles **13b** located in the end zone of the fermenter **3** are connected via pump **13a** and pipeline to the head **13c** located at biogas exit **14**.

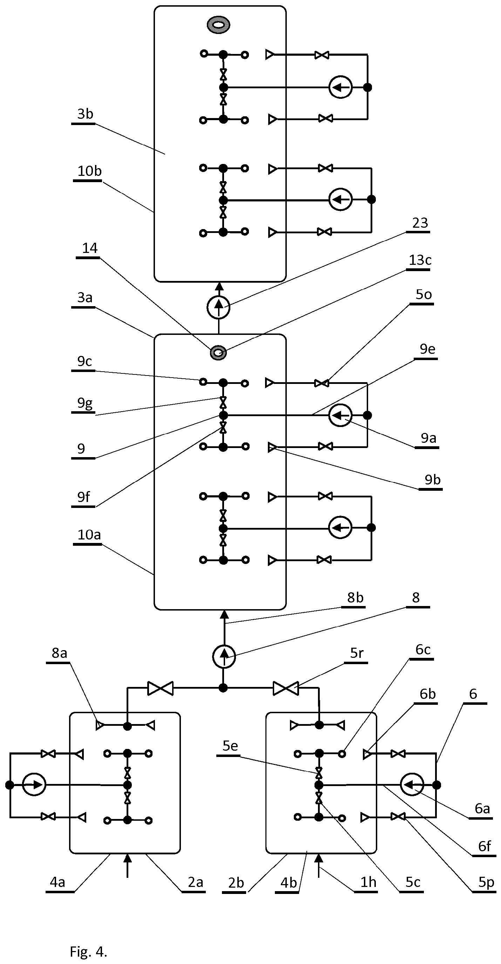

Figure 4 shows the arrangement of the tanks including biomass transfer and heating equipment, comprising two hydrolysers **2a** and **2b** arranged in parallel and connected to two fermenters **3a** and **3b** arranged in series. The hydrolyser tanks **4a** and **4b** are connected to slurry pipelines **1h**. At their ends, the tanks **4a** and **4b** are equipped with suction nozzles **8a** located at the bottom of each tank **4a** and **4b**, situated transversally with respect to the axes of tanks **4a** and **4b** and connected via valves **5r** as well as pump **8** and pipeline with a siphon **8b** to the tank **10a** of the fermenter **3a**. The end of the tank **3a** is connected via pipeline and pump **23** to the beginning of the tank **10b** of the second fermenter **3b**. The tanks **4a** and **4b** have mixing units **6** each equipped with two pairs of jet nozzles **6c** disposed along the axes of those tanks **4a** and **4b**, in their upper parts, whereas the jet nozzles **6c** are positioned transversally to their axes. The suction nozzles **6b** of the mixing units **6** are connected via pipelines and valves **5p** as well as via pumps **6a**, pipelines **6f** and control valves **5c** and **5e** to jet nozzles **6c**. Tanks **10a** and **10 b** have two segments **9** disposed along the axes of the tanks **10a** and **10b.** Jet nozzles **9c** arranged in pairs are positioned transversally with respect to the axes of the tanks **10a** and **10b** in their upper parts. The suction nozzles 9b of the fermenter mixing segment **9** are connected via pipelines and valves **5o** as well as via pump **9a,** pipeline **9e** and valves **9f** and **9g** to jet nozzles **9c.** Both tanks **10a** and **10b** have at their ends biogas exits **14,** in which there are placed scrubbing heads **13c.**

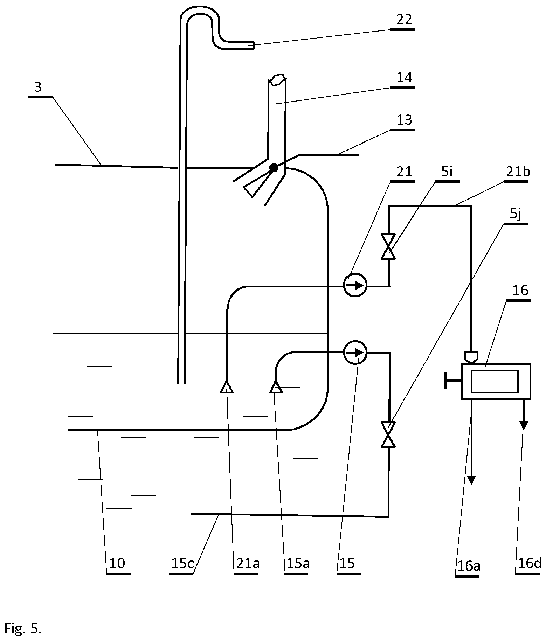

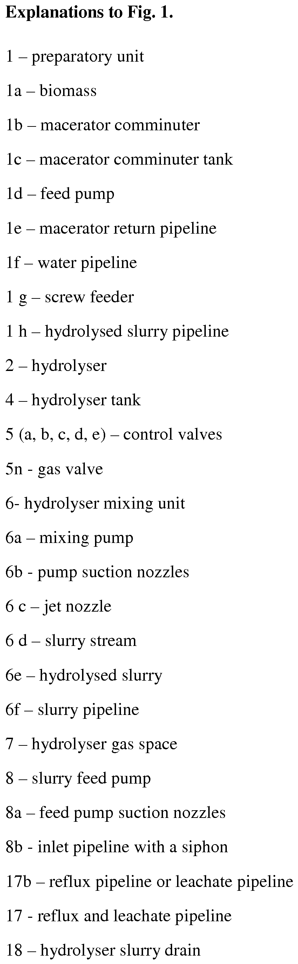

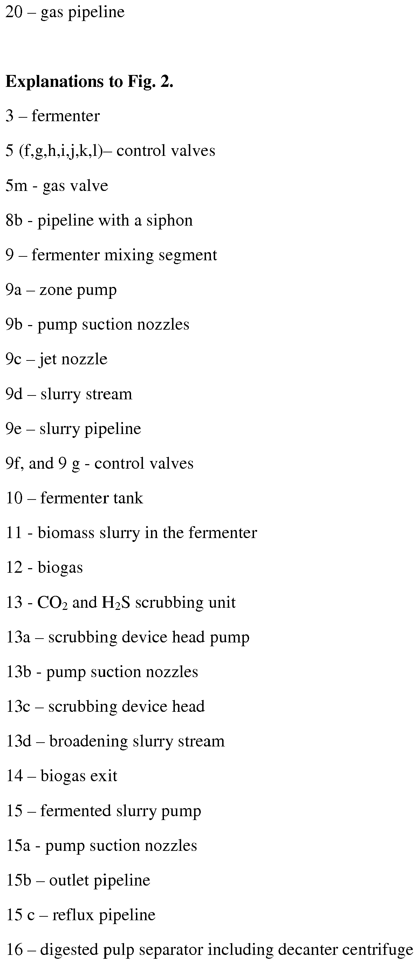

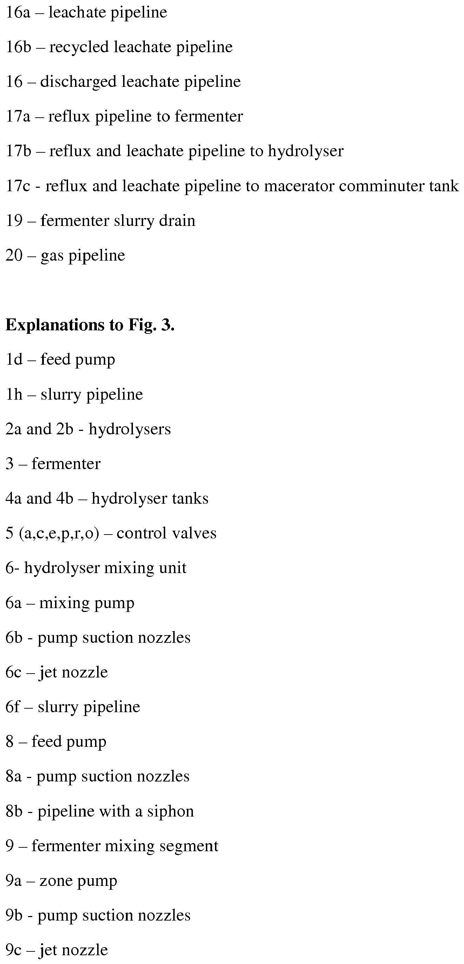

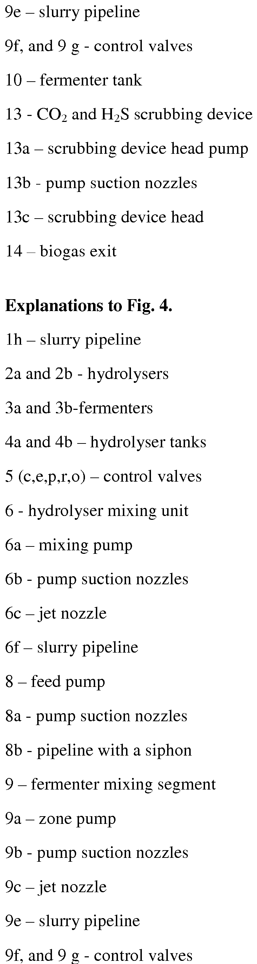

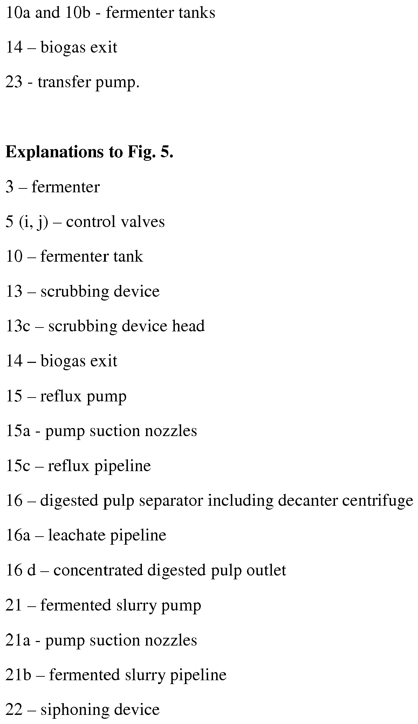

As shown in the drawing, Figure 5 shows a unit composed of two pumps **15** and **21** connected to the end part of the fermenter **3.** Reflux pump **15** is connected at one side to suction nozzles **15a** and, at the other side, it is connected through valve **5j** to reflux pipeline **15c.** Pump **21** is connected at one side to suction nozzles **21a** placed at the end of the tank **10** of the fermenter **3,** and, at the other side, it is connected through valve **5i** and pipeline **21b** to decanter centrifuge **16** which comprises a concentrated digested pulp outlet **16d** and a connection to leachate pipeline **16a.** The fermenter **3** is equipped with a siphoning device **22** to protect the tank **10** from excessive pressure.

## Claims

1. A method for transferring and mixing a biomass slurry in a hydrolyser by hydrolysing a biomass and transferring and mixing the hydrolysed slurry in a fermenter or fermenters connected in parallel using anaerobic processing of biomass into biogas, wherein biomass comminuted in a macerator comminuter, mixed with leachates from digested pulp separator, containing appropriate bacterial cultures, and again or repeatedly pumped through the macerator comminuter, **characterised in that** a biomass comminuted in a macerator comminuter with about 10% of biomass on the dry weight basis is mixed in the comminuter tank with a part of reflux and a slurry is passed again once or more times through the macerator comminuter, and then the comminuted slurry is pumped cyclically to a horizontal hydrolyser tank until it is filled at 50-70%, after which the biomass slurry is subjected to mixing in a single zone or in subsequent zones of the hydrolyser using slurry streams produced by jet nozzles, next, the hydrolysed slurry is pumped in portions to a fermenter whose bottom is sloping at an angle from 0,3% to 0,9% toward the outlet of the slurry, thus causing horizontal movement of the slurry during which it is subjected to periodical mixing in subsequent fermenter zones, counting from the first zone up to the penultimate zone, by slurry streams produces by jet nozzles, whereas in the last fermenter zone in the area of biogas discharge the biomass is continuously introduced in form of a broadening stream, and the fermented biomass after exiting the fermenter is split into two streams, the first one as a reflux is fed to the fermenter tank, the hydrolyser tank and the tank of the macerator comminuter, while the other stream of the fermented biomass is fed to a digested pulp separator from where a part of leachates is recycled back via a pipeline to the tank of the macerator mill and/or the hydrolyser tank, and biogas flowing out from the fermenter is treated and further processed in a known way.

2. Method according to claim 1, **characterised in that** the biomass slurry to be ejected from the jet nozzles is sucked at the bottom of the hydrolyser within that zone in which the respective jet nozzle is located, and, additionally, the biomass stream introduced at the top is directed vertically to the surface of the slurry in the hydrolyser tank.

3. Method according to claim 1, **characterised in that** the biomass slurry to be ejected by the jet nozzles is sucked at the bottom of the fermenter within the respective zone where the jet nozzle is located, and, additionally, the stream of biomass being fermented is directed vertically to the surface of the slurry in the hydrolyser tank.

4. Method according to claim 1, **characterised in that** in the course of hydrolysis and fermentation of biomass an equal elevated pressure of the gases in the hydrolyser and fermenter is maintained.

5. Method according to claim 1, **characterised in that** the biomass transfer and mixing processes are carried out in at least two hydrolysers arranged in parallel and in one or at least two fermenters arranged in parallel or in series.

6. Method according to claim 1, **characterised in that** before the biomass slurry is fed to hydrolyser nozzles, it is again comminuted in an attachment fitted in the transfer pump.

7. Method according to claim 1, **characterised in that** before the biomass slurry is fed to fermenter nozzles, it is again comminuted in an attachment fitted in the transfer pump.

8. A system for transferring and mixing of a biomass slurry in a hydrolyser and in a fermenter, consisting of macerator comminuter, hydrolyser, fermenter, digested pulp separator, as well as tanks, pipelines, valves and pumps, wherein a feeder is connected at the outlet of comminuter tank, through macerator comminuter, slurry pump and valve, with the tank at its inlet and to the tank there is connected the pipeline with leachates and water pipeline, and the pump is connected to the hydroliser **characterised in that** hydrolyser (**2**) is equipped with a slurry hydraulic mixing unit (**6**), wherein hydrolyser (**2**) at its end is connected through a pump (**8**) equipped with suction nozzles (**8a**) and a pipeline with a siphon (**8b**) to the tank (**10**) of the fermenter (**3**) whose bottom is sloping at an angle from 0,3% to 0,9% toward the outlet of the slurry, where said fermenter (**3**)_has at least two slurry mixing segments (**9**), and at the biogas exit (**14**) located at the top of the end part of the tank (**10**) there is installed a head (**13c**) of a scrubbing device (**13**), and additionally, the end part of the tank (**10**) is equipped with suction nozzles (**15a**) placed transversally with respect to the axis of the tank (**10**) connected to a pump (**15**) from which there run two pipeline branches (**15b**) and (**15c**), one branch (**15b**) connecting the fermenter (**3**) to a digested pulp separator (**16**) and the other branch (**15c**) connecting the fermenter (**3**) to the beginning of the tank (**10**) of the fermenter (**3**), to the initial part of the tank (**4**) of the hydrolyser (**2**) and to the tank (**1c**).

9. The system according to claim 8, **characterised in that** the hydrolyser mixing unit (**6**) comprises a mixing pump (**6a**) equipped with a comminuting attachment, one side of said pump connected to suction nozzles (**6b**) located at the bottom of the hydrolyser (**2**) transversally with respect to the axis of the hydrolyser tank (**4**), and the other side of the pump (**6a**) is connected to a valve (**5c**) or to a plurality of valves (**5c**) subsequently disposed along the hydrolyser (**2**), and each of the valves (**5c)** is connected to one jet nozzle (**6c**) or at least two jet nozzles (**6c**) located in the upper part of the hydrolyser (**2**).

10. The system according to claim 8, **characterised in that** fermenter mixing segments (**9**) comprise batteries composed of at least two jet nozzles (**9c**), positioned transversally and/or longitudinally in relation to the axis of the tank (**10**), placed in the upper part of the fermenter (**3**) and connected via pumps (**9a**) with comminuting attachments to suction nozzles (**9b**) placed at the bottom of the tank (**10**) within the respective zone.

11. The system according to claim 8, **characterised in that** the mixing segments (**9**) are disposed along the axis of the fermenter (**3**), one after another, in subsequent mixing zones.

12. The system according to claim 8, **characterised in that** the head (**13c**) of the scrubbing device (**13**) installed at biogas exit (**14**) from the tank (**10**) is connected by a pump (**13a**) to suction nozzles (**13b**) placed in the end part of the fermenter (**3**) transversally with respect to the axis of the tank (**10**).

13. The system according to claim 8, **characterised in that** the pump (**15**) is connected at one side to suction nozzles (**15a**) placed at the end of the fermenter (**3**) and, at the other side, it is connected through a valve (**5i**) and a pipeline (**15b**) to the digested pulp separator (**16**) and is connected via valve (**5j**) and pipeline (**15c**), valve (**5g**) and pipeline (**17c**) and through valve (**5d**) to tank (**1c**), and also through valve (**5h**) and pipeline (**17b**) to the tank (**4**) of the hydrolyser (2), and through valve (**5f**) and pipeline (**17a**) to the beginning of the tank (**10**) of the fermenter (**3**).

14. The system according to claim 13, **characterised in that** the pump (**15**) is connected via valve (**5j**) to pipeline (**15c**), and the pump (21) is connected at one side to the suction nozzles (**21a**) located at the end of the tank (**10**), and at the other end via valve (**21b**) and pipeline (**21c**) to the digested pulp separator (**16**).

15. The system according to claim 8, **characterized in that** the tank (**4**) of the hydrolyser (**2**) is connected through a conduit (**20**) to the tank (**10**) of the fermenter (**3**), whereas the tank (**10**) is equipped with a siphon (**22**) protecting the tanks from excess pressure.

## Patentansprüche

1. Verfahren zum Transportieren und Mischen einer Biomasse-Suspension in einem Hydrolyseur unter Anwendung der Biomasse-Hydrolyse sowie zum Transportieren und Mischen der hydrolysierten Suspension in einem Fermenter oder in den parallel verbundenen Fermentern unter Anwendung der anaeroben Aufbereitung von Biomasse zu Biogas, wobei die in einem Nasszerkleinerer zerkleinerte Biomasse mit Ablauf aus dem Nachgärungsabscheider, der entsprechende Bakterienkulturen enthält, vermischt und erneut oder mehrmals durch den Nasszerkleinerer geleitet wird, **gekennzeichnet dadurch, dass** die durch den Nasszerkleinerer zerkleinerte Biomasse im Zerkleinerertank mit einem Teil des Rückflusses vermischt und die Biomasse-Suspension erneut oder mehrmals durch den Nasszerkleinerer geleitet wird, und dann die zerkleinerte Suspension zyklisch in den horizontal angeordneten Hydrolyseurtank gepumpt wird, bis dieser zu 50 - 70% gefüllt ist, wonach die Biomasse-Suspension in der einzelnen Zone oder in nachfolgenden Zonen des Hydrolyseurs mit durch die Ausstoßdüsen erzeugten Suspensionsströmen vermischt wird, und dann die hydrolysierte Suspension portionsweise in den Fermenter gepumpt wird, wobei der Boden zwischen 0,3° und 0,9° schräg in Richtung des Suspensionsauslaufs geneigt ist, wodurch der Suspension eine horizontale Bewegung verliehen wird, bei der sie in den nachfolgenden Zonen des Fermenters beginnend von der ersten Zone bis zu der vorletzten Zone durch die von den Ausstoßdüsen erzeugten Suspensionsströme periodisch gemischt wird, während in der letzten Zone des Fermenters, im Bereich des Biogasauslaufs, die Biomasse-Suspension kontinuierlich in Form eines divergenten Stroms eingeleitet wird, und die gegorene Biomasse wird nach dem Verlassen des Fermenters in zwei Ströme geteilt - der erste Strom in Form von Rückfluss wird in den Fermentertank, in den Hydrolyseurtank und in den Nasszerkleinerertank geleitet, und der zweite Strom der gegorenen Biomasse wird in den Abscheider des Nachfermenters geleitet, wovon ein Teil des Ablaufs über eine Rohrleitung in den Nasszerkleinerertank und/oder in den Hydrolyseurtank zurückgeführt wird und das aus dem Fermenter kommende Biogas auf bekannte Weise gereinigt und weiterverarbeitet wird.

2. Das Verfahren nach dem Anspruch 1 **gekennzeichnet dadurch, dass** die durch die Ausstoßdüsen ausströmende Biomasse-Suspension am Boden des Hydrolyseurs im Bereich der Ausstoßdüse angesaugt und außerdem der von oben eingeführte Biomassestrom senkrecht zur Oberfläche der Suspension im Hydrolyseurtank geleitet wird.

3. Das Verfahren nach dem Anspruch 1 **gekennzeichnet dadurch, dass** die durch die Ausstoßdüsen ausströmende Biomasse-Suspension am Boden des Fermenters im Bereich der Ausstoßdüse angesaugt und außerdem der von oben eingeführte Strom der gärenden Biomasse senkrecht zur Oberfläche der Suspension im Hydrolyseurtank geleitet wird.

4. Das Verfahren nach dem Anspruch 1 **gekennzeichnet dadurch, dass** während der Hydrolyse und Fermentation von Biomasse der Druck der Gase im Hydrolyseur und im Fermenter gleich bleibt.

5. Das Verfahren nach dem Anspruch 1 **gekennzeichnet dadurch, dass** der Transport und die Mischung der Biomasse-Suspension in mindestens zwei parallel angeordneten Hydrolyseuren und in einem oder mindestens in zwei in Reihe oder parallel angeordneten Fermentern erfolgt.

6. Das Verfahren nach dem Anspruch 1 **gekennzeichnet dadurch, dass** die Biomasse-Suspension vor der Zuführung zu den Hydrolyseurdüsen in einem in der Förderpumpe montierten Zerkleinerungsaufsatz erneut zerkleinert wird.

7. Das Verfahren nach dem Anspruch 1 **gekennzeichnet dadurch, dass** die Biomasse-Suspension vor der Zuführung zu dem Fermenter in einem in der Förderpumpe montierten Zerkleinerungsaufsatz erneut zerkleinert wird.

8. Das System zum Transport und Mischen der Biomasse-Suspension im Hydrolyseur und im Fermenter, bestehend aus einem Nasszerkleinerer, Hydrolyseur, Fermenter, Nachgärungsabscheider, aus Tanks, Rohrleitungen, Ventilen und Pumpen, wobei die Zuführung am Auslauf des Zerkleinerertanks über den Nasszerkleinerer und die Pumpe sowie ein Ventil mit dem Eingang zu diesem Tank verbunden ist, und außerdem ist der Tank mit der Leitung des Ablaufs sowie einer Wasserleitung verbunden, die Pumpe dagegen ist auch mit dem Hydrolyseur verbunden, **gekennzeichnet dadurch, dass** der Hydrolyseur (**2**) mit einer Einheit (**6**) zur hydraulischen Mischung der Suspension ausgestattet ist, wobei der Hydrolyseur (**2**) am Ende über eine Pumpe (**8**), die mit Saugdüsen **8a** und einer Rohrleitung mit Siphon (**8b**) ausgestattet ist, mit dem Tank (**10**) des Fermenters (**3**) verbunden ist, dessen Boden unter dem Winkel von 0,3° bis 0,9° schräg in Richtung des Suspensionsauslaufs geneigt ist, wobei der Fermenter (**3**) mindestens zwei Segmente (**9**) zum Suspensionsmischen aufweist und am Biogasauslass (**14**), der sich oben am Ende des Tanks (**10**) befindet, ist der Kopf (**13c**) einer Auswaschvorrichtung (**13**) installiert, darüber hinaus ist das Ende des Tanks (**10**) mit Saugdüsen (**15a**) ausgestattet, die quer zur Achse des Tanks (**10**) angeordnet und mit der Pumpe (**15**) verbunden sind, von der zwei Abzweigungen (**15b**) und (**15c**) herausgeführt werden, durch die Abzweigung (**15b**), die den Fermenter (**3**) mit dem Nachgärungsabscheider (**16**) verbindet, und die Abzweigung (**15c**), die den Fermenter (**3**) mit dem Anfangsteil des Tanks (**10**) des Fermenters (**3**), mit dem Anfangsteil des Tanks (**4**) des Hydrolyseurs (**2**) und mit dem Tank (**1c**) verbindet.

9. Das System nach dem Anspruch 8 **gekennzeichnet dadurch, dass** die Mischeinheit (**6**) des Hydrolyseurs eine Mischpumpe (**6a**) mit einem Zerkleinerungsaufsatz aufweist, der auf einer Seite mit Saugdüsen (**6b**), die am Boden des Hydrolyseurs (**2**)quer zur Achse des Tanks (**4**) angeordnet sind, verbunden ist, während die Pumpe (**6a**) auf der anderen Seite mit einem Ventil (**5c**) oder mit Ventilen (**5c**) verbunden ist, die nacheinander entlang des Hydrolyseurs (**2**) angeordnet sind, und jedes der Ventile (**5c**) ist mit der Ausstoßdüse (**6c**) oder mit mindestens zwei Ausstoßdüsen (**6c**) verbunden, die sich im oberen Teil des Hydrolyseurs (**2**) befinden.

10. Das System nach dem Anspruch 8 **gekennzeichnet dadurch, dass** die Mischungssegmente (**9**) des Fermenters Anordnungen aufweisen, die aus mindestens zwei Ausstoßdüsen (**9c**) bestehen, die quer und/oder längs zur Achse des Tanks (**10**) angeordnet, im oberen Teil des Fermenters (**3**) platziert sind und über Pumpen (**9a**) mit Zerkleinerungsaufsätzen mit am Boden des Tanks (**10**) innerhalb des jeweiligen Bereichs angeordneten Saugdüsen (**9b**) verbunden sind.

11. Das System nach dem Anspruch 8 **gekennzeichnet dadurch, dass** die Mischungssegmente (**9**) längs der Achse des Fermenters (**3**) ein nach dem anderen in nacheinander folgenden Mischungszonen angeordnet sind.

12. Das System nach dem Anspruch 8 **gekennzeichnet dadurch, dass** der auf dem Biogasausgang (**14**) des Tanks (**10**) installierte Kopf (**13c**) der Auswaschvorrichtung (**13**) über die Pumpe (**13a**) mit Saugdüsen (**13b**), die im Endteil des Fermenters (**3**) quer zu der Achse des Tanks (**10**) angeordnet sind, verbunden ist.

13. Das System nach dem Anspruch 8 **gekennzeichnet dadurch, dass** die Pumpe (**15**) auf einer Seite mit Saugdüsen (**15a**), die am Ende des Fermenters (**3**) platziert sind, verbunden ist, und auf der anderen Seite über das Ventil (**5i**) und die Rohrleitung (**15b**) mit Nachgärungsabscheider (**16**) des Fermenters verbunden ist und über das Ventil (**5j**) und die Rohrleitung (**15c**), über das Ventil (**5g**) und die Rohrleitung (**17c**), sowie über das Ventil (**5d**) mit dem Tank (**1c**) ein Verbindung aufweist, und auch über das Ventil (**5h**) und die Rohrleitung (**17b**) mit dem Tank (**4**) des Hydrolyseurs (**2**) und über das Ventil (**5f**) und die Rohrleitung (**17a**) mit dem Anfang des Tanks (**10**) des Fermenters (**3**).

14. Das System nach dem Anspruch 13 **gekennzeichnet dadurch, dass** die Pumpe (**15**) über das Ventil (**5j**) mit der Rohrleitung (**15c**) verbunden ist und die Pumpe (**21**) auf einer Seite mit den am Ende des Tanks (**10**) angeordneten Saugdüsen (**21a**) und auf der anderen Seite über das Ventil (**21b**) und die Rohrleitung (**21c**) mit dem Nachgärungsabscheider (**16**) verbunden ist.

15. Das System nach dem Anspruch 8 **gekennzeichnet dadurch, dass** der Tank (**4**) des Hydrolyseurs (**2**) mit dem Tank (**10**) des Fermenters (**3**) durch die Leitung (**20**) verbunden ist, wobei der Tank (**10**) mit einem Siphon (**22**) zum Schutz des Tanks gegen Überdruck ausgestattet ist.

## Revendications

1. Procédé de transport et de mélange de la suspension de biomasse dans l'hydrolyseur utilisant l'hydrolyse de la biomasse ainsi que de transport et de mélange de la suspension hydrolysée dans un fermenteur ou des fermenteurs connectés en parallèle avec l'utilisation de la conversion de la biomasse anaérobie en biogaz, dans lequel la biomasse étant broyée dans un broyeur - macérateur est mélangée avec l'effluent du séparateur de digestat contenant des cultures bactériennes appropriées, et étant passée à nouveau ou plusieurs fois dans le macérateur **caractérisé en ce que** la biomasse broyée par le broyeur - macérateur est mélangée dans le réservoir du broyeur avec une partie du reflux et la suspension de biomasse est passée à nouveau ou plusieurs fois dans le broyeur, puis la suspension broyée est pompée cycliquement dans un réservoir d'hydrolyseur disposé horizontalement jusqu'à ce qu'il soit remplie dans les 50 à 70%, puis la suspension de biomasse est soumise à un mélange dans une seule zone ou dans des zones d'hydrolyseur subséquentes avec des courants de suspension produits par des buses d'éjection, puis la suspension hydrolysée est pompée par portions dans un fermenteur à fond incliné selon un angle de 0,3° à 0,9° vers la sortie de la suspension, lui donnant un mouvement horizontal, dans laquelle il est périodiquement mélangé dans les zones de fermentation suivantes de la première zone à l'avant-dernière zone par les flux de suspension générés par les buses d'éjection, tandis que dans la dernière zone de fermentation, dans la région de sortie du biogaz, une suspension de biomasse sous la forme d'un flux divergent est introduite en continu, et la biomasse fermentée après avoir quitté le fermenteur est divisée en deux flux - le premier flux sous forme de reflux est dirigé vers le réservoir du fermenteur, vers le réservoir d'hydrolyseur et vers le réservoir du broyeur - macérateur, et le deuxième flux de biomasse fermentée est dirigé vers le séparateur de digestats, dont une partie des effluents est renvoyée par pipeline au réservoir de broyeur - macérateur et/ou au réservoir d'hydrolyseur, et le biogaz s'écoulant du fermenteur est soumis à une purification et à un traitement ultérieur d'une manière connue.

2. Procédé selon la revendication 1, **caractérisé en ce que** la suspension de biomasse s'écoulant par les buses d'éjection est aspirée au fond de l'hydrolyseur dans la région de la zone où se trouve la buse d'éjection, et en outre le flux de biomasse introduit par le haut est dirigé perpendiculairement à la surface de suspension dans le réservoir d'hydrolyseur.

3. Procédé selon la revendication 1, **caractérisé en ce que** la suspension de biomasse s'écoulant par les buses d'éjection est aspirée au fond du fermenteur, dans la région de la zone dans laquelle se trouve la buse d'éjection, et en outre le flux de biomasse en introduit par le haut est dirigé perpendiculairement à la surface de suspension dans le réservoir d'hydrolyseur.

4. Procédé selon la revendication 1 **caractérisé en ce que** pendant l'hydrolyse et la fermentation de la biomasse, la même pression de gaz plus élevée est maintenue dans l'hydrolyseur et le fermenteur.

5. Procédé selon la revendication **caractérisé en ce que** le transport et le mélange de la suspension de biomasse sont effectués dans au moins deux hydrolyseurs disposés en parallèle et dans un ou au moins deux fermenteurs disposés en série ou en parallèle.

6. Procédé selon la revendication **caractérisé en ce que** avant d'amener la suspension de biomasse aux buses de l'hydrolyseur, elle est à nouveau raffinée dans un accessoire de broyage monté dans une pompe à pression.

7. Procédé selon la revendication 1, **caractérisé en ce que** avant d'amener la suspension de biomasse vers les buses de fermenteur, elle est soumise à un raffinement dans un accessoire de broyage monté dans une pompe à pression.

8. Le système de transport et de mélange de la suspension de biomasse dans l'hydrolyseur et le fermenteur, composé d'un broyeur - macérateur, hydrolyseur, fermenteur, séparateur de digestats, réservoirs, pipelines, vannes et pompes, dans lequel le chargeur à la sortie du réservoir du broyeur est connecté par le broyeur - macérateur et pompe, et soupape avec entrée dans ce réservoir, en outre, le réservoir est connecté aux pipelines de l'effluent et d'eau, et la pompe est également connectée à un hydrolyseur **caractérisé en ce que** l'hydrolyseur (**2**) est équipé d'un ensemble (**6**) pour le mélange hydraulique de la suspension, dans lequel l'hydrolyseur (2) à son fin étant relié par une pompe (**8**) équipée de ventouses (**8a**) et d'un pipeline de siphon (**8b**) avec réservoir (**10**) du fermenteur (**3**), dont le fond est incliné d'un angle de 0,3° à 0,9° vers la sortie de la suspension, dans lequel l'hydrolyseur (**3**) comportant au moins deux segments (**9**) pour mélanger la suspension, tandis que la sortie de biogaz (**14**) située en haut dans la partie d'extrémité du réservoir (**10**) la tête (**13c**) du dispositif de lavage (**13**) est installée, la partie d'extrémité du réservoir (**10**) est pourvue de ventouses (**15a**) situées transversalement à l'axe du réservoir (**10**) reliées à la pompe (**15**), à partir desquelles deux branches (**15b**) et (**15c**), sont conduites par la branche de raccordement (**15b**) du fermenteur (**3**) avec le séparateur (**16**) de digestat et la branche (**15c**) reliant le fermenteur (**3**) à la partie de départ de la réservoir (**10**) de fermenteur (**3**), avec la partie de départ du réservoir (**4**) d'hydrolyseur (**2**) et du réservoir (**1c**).

9. Système selon la revendication 8, **caractérisé en ce que** l'ensemble de mélange d'hydrolyseur (**6**) comporte une pompe de mélange (**6a**) avec un accessoire de broyage relié d'un côté aux ventouses (**6b**) situées au fond de l'hydrolyseur (**2**) transversalement à l'axe du réservoir (**4**) tandis que d'autre part la pompe (**6a**) est connectée à la soupape (**5c**) ou aux soupapes (**5c**) disposées le long de l'hydrolyseur (**2**), et chacune des soupapes (**5c**) est reliée à la buse d'éjection (**6c**) ou à au moins deux buses d'éjection (**6c**), placées au-dessus de l'hydrolyseur (**2**).

10. Système selon la revendication 8, **caractérisé en ce que** les segments (**9**) de mélange du fermenteur comportent des ensembles composés d'au moins deux buses d'éjection (**9c**), disposées transversalement et/ou longitudinalement par rapport à l'axe du réservoir (**10**), situées dans la partie supérieure du fermenteur (**3**) et reliées par des pompes (**9a**) avec accessoires de broyage, avec des ventouses (**9b**) situées au fond du réservoir (**10**) dans la zone donnée.

11. Système selon la revendication 8, **caractérisé en ce que** les segments (**9**) de mélange sont situés le long de l'axe du fermenteur (3), l'un après l'autre, dans des zones de mélange successives.

12. Système selon la revendication 8, **caractérisé en ce que** la tête (**13c**) du dispositif de lavage (**13**) installé à la sortie (**14**) du biogaz du réservoir (**10**) est reliée par une pompe (**13a**) à ventouses (**13b**) situées à l'extrémité du fermenteur (**3**) transversalement à l'axe réservoir (**10**).

13. Système selon la revendication 8, **caractérisé en ce que** la pompe (**15**) est connectée d'un côté avec des ventouses (**15a**) situées à l'extrémité du fermenteur (**3**), et d'autre part connectée via une vanne (**5i**) et un pipeline (**15b**) au séparateur de digestat (**16**) et a une connexion par une vanne (**5j**) et un pipeline (**15c**), une vanne (**5g**) et un pipeline (**17c**) et par une vanne (**5d**) avec un réservoir (**1c**), ainsi que via une vanne (**5h**) et un pipeline (**17b**) avec un réservoir (**4**) de l'hydrolyseur (**2**) et par la vanne (**5f**) et un pipeline (**17a**) avec le début de la réservoir (**10**) (**3**).

14. Système selon la revendication **13**, **caractérisé en ce que** la pompe (**15**) est reliée via une valve (**5j**) au pipeline (**15c**) et la pompe (**21**) est reliée d'un côté à des ventouses (**21a**) situées à l'extrémité du réservoir (**10**) et de l'autre vanne (**21b**) et pipeline (**21c**) avec séparateur (**16**) de digestat.

15. Système selon la revendication 8, **caractérisé en ce que** le réservoir (**4**) de l'hydrolyseur (**2**) relié par un conduit (**20**) au réservoir (**10**) du fermenteur (**3**), lequel réservoir (**10**) étant équipé d'un siphon (**22**) protégeant les réservoirs contre les pressions excessives.
